# EUROPEAN PATENT APPLICATION

(11) **EP 4 238 611 A1**
(43) Date of publication of application: **06.09.2023**
(21) Application number: 21886020.3
(22) Date of filing: 20.10.2021
(51) Int. Cl.: A61P 15/08, A61K 36/638, A61K 36/77, A61K 36/815, A23L 33/105

(54) **COMPOSITION FOR PREVENTING, IMPROVING, OR ALLEVIATING MENSTRUAL SYMPTOMS**

(30) Priority: 28.10.2020 JP 2020180446
(71) Applicant: SUMITOMO CHEMICAL COMPANY, LIMITED, Tokyo 103-6020 (JP)
(72) Inventor: SASAKI, Katsunori, Tokyo 104-8260 (JP); HIRANO, Keiko, Tokyo 104-8260 (JP); YOSHIDA, Takamitsu, Nagoya-shi, Aichi 466-0054 (JP); MAEDA, Susumu, Nagoya-shi, Aichi 466-0054 (JP); KITAMAKI, Yuki, Nagoya-shi, Aichi 466-0054 (JP); SHIMATO, Yota, Nagoya-shi, Aichi 466-0054 (JP); YANASE, Mari, Nagoya-shi, Aichi 466-0054 (JP)
(74) Representative: J A Kemp LLP
(86) International application number: PCT/JP2021/038725
(87) International publication number: WO 2022/091901

(57) **Abstract**

Component (1): at least one kind of herbal medicine selected from the group consisting of Longan aril, common fenugreek seed, Lycium fruit, songaria cynomorium herb, black sesame, Dipsacus japonicus root, Drynaria herb, flatstem milkvetch seed, black bean, stink-bug, sea horse and donkey glue

Component (2): at least one kind of herbal medicine selected from the group consisting of Ligustrum fruit, oyster shell, yerbadetajo herb, mulberry fruit, cassia seed, mentha herb, mulberry leaf, gardenia fruit, green tea leaf, tangle weed, Chrysanthemum indicum L., Oldenlandia herbacea, plantago seed, plantago herb, wheat seed, feather cockscomb seed, chrysanthemum flower, dandelion herb, Isatis tinctoria L., barbated skullcup herb, snowbellleaf tickclover herb, turmeric, garden burnet root, and Crocus sativus

Component (3): at least one kind of herbal medicine selected from the group consisting of Curcuma rhizome, Suberect spatholobus stem, wild turmeric, Sparganium rhizome, Luffa aegyptica Mill., Carthamus tinctorius, Artemisia anomala, Hairyveine agrimonia herb, Fortune windmillpalm petiole, Dalbergia odorifera, dragon's blood, leech, Panax notoginseng root, Aizoon stonecrop herb, Ophicalcitum, and lotus root node

## Description

### TECHNICAL FIELD

The present invention relates to a composition for use in preventing, improving, or alleviating menstrual symptoms, which includes a herbal medicine component.

### BACKGROUND ART

There are three types of symptoms associated with menstruation, that is, menstrual symptoms of a symptom that occurs before menstruation (premenstrual symptom), a symptom that occurs during menstruation (symptom during menstruation), and a symptom that occurs from the premenstrual period to the menstrual period. According to the preceding studies, it has been reported that "referring to the surveys since 2000, premenstrual symptoms were 73.9 to 87.7%, symptoms during menstruation were 79 to 98.5%, symptoms from before menstruation to during menstruation were 98.4 to 99.9%, and problems in daily life were 27.1 to 35.3%". Further, the socioeconomic loss due to the menstrual symptoms is estimated to be as much as 682.8 billion yen per year, and it is not only a serious problem in daily life and social life for women, but also a social problem that is strongly desired to be solved (Non-Patent Document 1).

Drug therapy with over-the-counter or prescription drug is generally used to treat menstrual symptoms, but from the viewpoint of optimizing the health care cost, it is desirable to try to prevent, improve, or alleviate the menstrual symptoms by a food composition. For example, Patent Document 1 discloses as a food for alleviating period pain (menstrual pain) that is one of the menstrual symptoms, a period-pain relief food obtained by using Mitchella repens and Rubus indaeus as the main components, and adding flavoring components such as amino acids and vitamin C, a sweetening component such as fructose or xylitol, and a particle size-regulating/shape-retaining component thereto.

Herbal medicines belong to a group of medicinal plants used in pharmaceuticals or foods in Chinese medicine that is a representative traditional medicine system and in Kampo medicine derived from the Chinese medicine, and the efficacy and usage of such herbal medicines are systematized based on the clinical experience accumulated over thousands of years. Further, in the idea of presymptomatic state taken root in Chinese medicine, affinity for prevention is high, and expectations for a food material that realizes QOL improvement through the prevention are growing. However, there are still many unexplained aspects of the expression of the efficacy by the combinations of herbal medicines, and there is more than enough room to find new combinations and to construct beneficial food compositions.

### PRIOR ART DOCUMENTS

### PATENT DOCUMENTS

Patent Document 1: JP-A-2004-121174

### NON-PATENT DOCUMENT

Non-Patent Document 1: Doshisha ClinicalPsychology:Therapy and Research (2014) Vol. 4, No. 1, Pp. 25-37

### SUMMARY OF THE INVENTION

### PROBLEMS TO BE SOLVED BY THE INVENTION

An object of the present invention is to provide a novel composition including a herbal medicine component, which can be used for preventing, improving, or alleviating menstrual symptoms.

### MEANS FOR SOLVING THE PROBLEMS

As a result of intensive studies to achieve the above object, the present inventors have found that it is possible to prevent, improve, or alleviate menstrual symptoms by selecting and mixing three groups of herbal medicines having specific medicinal benefit coverage and nature taste. The present invention has been completed through further investigation based on this finding, and provides a novel composition including a herbal medicine component.

That is, the present invention is as follows.
[1] A composition, including as active components the following components (1), (2), and (3):
   component (1): at least one kind of herbal medicine selected from the group consisting of Longan aril, common fenugreek seed, Lycium fruit, songaria cynomorium herb, black sesame, Dipsacus japonicus root, Drynaria herb, flatstem milkvetch seed, black bean, stink-bug, sea horse, and donkey glue;
   component (2): at least one kind of herbal medicine selected from the group consisting of Ligustrum fruit, oyster shell, yerbadetajo herb, mulberry fruit, cassia seed, mentha herb, mulberry leaf, gardenia fruit, green tea leaf, tangle weed, Chrysanthemum indicum L., Oldenlandia herbacea, plantago seed, plantago herb, wheat seed, feather cockscomb seed, chrysanthemum flower, dandelion herb, Isatis tinctoria L., barbated skullcup herb, snowbellleaf tickclover herb, turmeric, garden burnet root, and Crocus sativus; and
   component (3): at least one kind of herbal medicine selected from the group consisting of Curcuma rhizome, Suberect spatholobus stem, wild turmeric, Sparganium rhizome, Luffa aegyptica Mill., Carthamus tinctorius, Artemisia anomala, Hairyveine agrimonia herb, Fortune windmillpalm petiole, Dalbergia odorifera, dragon's blood, leech, Panax notoginseng root, Aizoon stonecrop herb, Ophicalcitum, and lotus root node.
[2] The composition described in [1], in which the component (2) is at least one kind of herbal medicine selected from the group consisting of Ligustrum fruit, oyster shell, yerbadetajo herb, mulberry fruit, and cassia seed.
[3] The composition described in [1] or [2], in which the composition is for use in preventing, improving, or alleviating menstrual symptoms.
[4] The composition described in any one of [1] to [3], in which the menstrual symptoms are at least one selected from the group consisting of menstrual pain, lower abdominal pain, breast strain or pain, abnormality in menstrual bleeding amount, abnormality in vaginal discharge, anemia, unusual increase in appetite, dyspeptic symptom, feeling of cold, swelling, headache, constipation, diarrhea, abdominal bloating, and symptoms in the genital area and the surrounding area thereof.
[5] The composition described in any one of [1] to [4], in which the composition is a food composition or a pharmaceutical composition.

### EFFECT OF THE INVENTION

According to the composition of the present invention, the whole menstrual symptoms can be prevented, improved, or alleviated, and in particular, menstrual pain, lower abdominal pain, breast strain or pain, abnormality in menstrual bleeding amount, abnormality in vaginal discharge, anemia, unusual increase in appetite, dyspeptic symptom, feeling of cold, swelling, headache, constipation, diarrhea, abdominal bloating, symptoms in the genital area and the surrounding area thereof, and the like can be prevented, improved, or alleviated. Thus, the composition of the present invention can contribute to the improvement of QOL and the solution of social problems.

Further, according to the composition of the present invention, at least one symptom selected from bad complexion/sallowness, unhealthy complexion, dull skin/dark circles under the eyes, acne/pimples, difficulty in healing wounds/bruises, rough skin/itchy skin, decreased libido, low back pain, menopausal symptom, chronic fatigue, hair loss, tinnitus, dry eye, eye fatigue, emotional lability, dysphoria/depressive symptom, sleepiness, difficulty in falling asleep, light sleep, ease of catching a cold, redness of the palm, and the like can be prevented, improved, or alleviated.

### MODE FOR CARRYING OUT THE INVENTION

Medicinal benefit coverage is one of the concepts used in Chinese medicine (TCM), refers to which part of the human body a herbal medicine acts on, and indicates the action site where the medicinal effect appears. The medicinal benefit coverage is composed of so-called "five organs", that is, "liver/heart/spleen/lung/kidney", and the five organs in Chinese medicine do not correspond to anatomical organs, but rather mean "functions" of the human body. The liver is related to the autonomic nerve, the emotion, the motor nerve, the gynecological system, and the liver, stores the blood, and controls the blood so that the life force and blood smoothly flow. The heart is related to the circulatory system including the brain and the heart, and has a function of circulating the blood throughout the body and nourishing the body. The spleen is related to the digestive system, allergies, and part of water metabolism, digests and absorbs foods and drinks, and creates "life force, blood, and colorless bodily fluids", which are the basics of the body. The lung is related to the respiratory system, the skin, part of water metabolism, and allergies, works together with the spleen to create "life force", spreads the life force throughout the body, regulates the flow of colorless bodily fluids, protects the body from external bad vibes, and is closely related to the immune system. The kidney is related to the reproductive system, the urinary system, the endocrine system, and aging and development, stores the source of vital energy, controls the water metabolism of the whole body, and produces and reabsorbs urine. In Chinese medicine, the five organs work together to generate and store the vital spirit of the body, and control the mental activity.

Nature taste (also referred to as "qi taste") is a type of classification system that indicates a nature of a herbal medicine, each taste is related to the five organs, and the taste itself has its own role. Most commonly, the nature taste indicates a nature of cooling and warming the human body by the five natures of "cold/cool/flat/warm/heat", and the five tastes of "hot/sweet/acid/bitter/salty" indicate the taste at the time of ingestion and are also indicators of the efficacy. In the five tastes, "light/astringent" may also be added in some cases. The "heat" and "warm" have a function of warding off cold, warming the body, stimulating the body, and activating the body. In addition, the "cold" and "cool" have a function of cooling the body heat, detoxifying, and calming the mind, and the "flat" refers to a nature of neither warming nor cooling. The "cold" may be further divided into "great cold", "cold", and "slight cold" depending on the degree. The "hot" is related to the lung, and has an effect of sweating, circulating the life force, and diverting oneself. The "sweet" is related to the spleen, and has a supplementing or alleviating effect. The "acid" is related to the liver, and has a tightening effect. The "bitter" is related to the heart, and has an effect of cooling the heat, drying the dampness, and lowering the dampness. The "salty" is related to the kidney, and has an effect of softening hard things, and moistening and lowering them. The nature taste may be subdivided into "great cold", "slight bitter" and the like depending on the degree.

The medicinal benefit coverage and nature taste are set for almost all the herbal medicines, herbal medicines that are coincident in the medicinal benefit coverage and/or the nature taste tend to have the same efficacy. The medicinal benefit coverage and nature taste are representatives of technical ideas that help to systematize the efficacy/prescription design of a huge number of herbal medicines.

The composition of the present invention includes components (1), (2), and (3) as the active components. Components (1), (2), and (3) belong to a group of herbal medicines each having the common medicinal benefit coverage and/or nature taste as shown in Table 1. For each herbal medicine, the generally known origin and site are used.

Component (1) is a herbal medicine having a medicinal benefit coverage of "liver" or "heart" and a nature of "warm" or "flat" in the five natures, and is expected to have an effect of increasing the blood. Specifically, the component (1) is at least one kind selected from the group consisting of Longan aril, common fenugreek seed, Lycium fruit, songaria cynomorium herb, black sesame, Dipsacus japonicus root, Drynaria herb, flatstem milkvetch seed, black bean, stink-bug, sea horse, and donkey glue. Among these herbal medicines, from the viewpoint of the availability (supply stability), the taste, or the efficacy, at least one kind selected from the group consisting of Longan aril, common fenugreek seed, Lycium fruit, songaria cynomorium herb, black sesame, Dipsacus japonicus root, Drynaria herb, and flatstem milkvetch seed is preferable, and from the viewpoint of exerting the effect with a small amount of raw material herbal medicine, at least one kind selected from the group consisting of Longan aril, common fenugreek seed, Lycium fruit, and songaria cynomorium herb is more preferable, and Longan aril is particularly preferable. The component (1) may be one kind of herbal medicine, two kinds of herbal medicines, three kinds of herbal medicines, or four or more kinds of herbal medicines.

Component (2) is a herbal medicine having a medicinal benefit coverage of "liver" and a nature of "cold" or "cool" in the five natures. Specifically, component (2) is at least one kind selected from the group consisting of Ligustrum fruit, oyster shell, yerbadetajo herb, mulberry fruit, cassia seed, mentha herb, mulberry leaf, gardenia fruit, green tea leaf, tangle weed, Chrysanthemum indicum L., Oldenlandia herbacea, plantago seed, plantago herb, wheat seed, feather cockscomb seed, chrysanthemum flower, dandelion herb, Isatis tinctoria L., barbated skullcup herb, snowbellleaf tickclover herb, turmeric, garden burnet root, and Crocus sativus. The component (2) may be one kind of herbal medicine, two kinds of herbal medicines, three kinds of herbal medicines, or four or more kinds of herbal medicines.

As the component (2), among the above-described herbal medicines, at least one kind selected from the group consisting of Ligustrum fruit, oyster shell, yerbadetajo herb, mulberry fruit, and cassia seed, having medicinal benefit coverages of "liver" and "kidney" is preferable, at least one kind selected from the group consisting of Ligustrum fruit, oyster shell, yerbadetajo herb, and cassia seed is more preferable from the viewpoint of exerting the effect with a small amount of raw material herbal medicine, and Ligustrum fruit and/or oyster shell are particularly preferable from the viewpoint of the taste or the efficacy.

Further, as the component (2), at least one kind selected from the group consisting of cassia seed, mentha herb, plantago seed, and plantago herb is preferable, and when the composition of the present invention contains at least one of these components, the intestine regulating function of these herbal medicines can be enhanced and exerted, and thus, it is possible to obtain a composition with which a characteristic effect can be experienced in a short period of time.

Further, as the component (2), at least one kind selected from the group consisting of oyster shell, mentha herb, green tea leaf, Oldenlandia herbacea, plantago seed, plantago herb, barbated skullcup herb, and snowbellleaf tickclover herb is preferable, and when the composition of the present invention contains at least one of these components, the metabolism-improving effect of these herbal medicines can be enhanced and exerted, and thus, it is possible to obtain a composition with which a characteristic effect can be experienced in a short period of time.

Component (3) is a herbal medicine having a medicinal benefit coverage of "liver" or "heart" and a nature of "warm" or "flat" in the five natures, and is expected to have an effect of activating the circulation of blood. Specifically, the component (3) is at least one kind selected from the group consisting of Curcuma rhizome, Suberect spatholobus stem, wild turmeric, Sparganium rhizome, Luffa aegyptica Mill., Carthamus tinctorius, Artemisia anomala, Hairyveine agrimonia herb, Fortune windmillpalm petiole, Dalbergia odorifera, dragon's blood, leech, Panax notoginseng root, Aizoon stonecrop herb, Ophicalcitum, and lotus root node. Among these herbal medicines, from the viewpoint of the taste or the efficacy, at least one kind selected from the group consisting of Curcuma rhizome, Suberect spatholobus stem, wild turmeric, Sparganium rhizome, Luffa aegyptica Mill., Carthamus tinctorius, Artemisia anomala, Hairyveine agrimonia herb, and Fortune windmillpalm petiole is preferable, and at least one kind selected from the group consisting of Curcuma rhizome, Suberect spatholobus stem, wild turmeric, Sparganium rhizome, and Luffa aegyptica Mill. Is more preferable, and from the viewpoint of exerting the effect with a small amount of raw material herbal medicine, Curcuma rhizome, Suberect spatholobus stem, and wild turmeric are particularly preferable. The component (3) may be one kind of herbal medicine, two kinds of herbal medicines, three kinds of herbal medicines, or four or more kinds of herbal medicines.

The medicinal benefit coverages and five natures of the herbal medicines of components (1), (2), and (3) are collectively shown in Table 1 below.

**[Table 1]**

| | Herbal medicine name | Medicinal benefit coverage | Five natures | | Herbal medicine name | Medicinal benefit coverage | Five natures |
|---|---|---|---|---|---|---|---|
| | Longan aril | Heart / spleen | Flat | | Ligustrum fruit | Liver / kidney | Sweet / bitter / cool |
| | Common fenugreek seed | Liver / kidney | Great warm | | Oyster shell | Liver / gall bladder / kidney | Salty / astringent, slight cold |
| | Lycium fruit | Liver / kidney / lung | Flat | | Yerbadetajo herb | Liver / kidney | Sweet / acid, cold |
| | Songaria cynomorium herb | Liver / kidney | Warm | | Mulberry fruit | Heart / liver / kidney | Sweet, cold |
| | Black sesame | Liver / kidney / lung / spleen | Flat | | Cassia seed | Liver / gall bladder / kidney | Sweet / bitter / salty, slight cold |
| | Dipsacus japonicus root | Liver / kidney | Great warm | | Mentha herb | Lung / liver | Hot, cool |
| | Drynaria herb | Liver / kidney | Warm | | Mulberry leaf | Lung / liver | Bitter / sweet, cold |
| Component (1) | Flatstem milkvetch seed | Liver / kidney | Warm | | Gardenia fruit | Heart / lung / liver / stomach / triple warmer | Bitter, cold |
| | Black bean | Liver / kidney | Flat | | Green tea leaf | Heart / lung / liver / kidney / spleen / stomach | Bitter / slight sweet, slight cold |
| | Stink-bug | Liver / kidney / spleen | Warm | Component (2) | Tangle weed | Liver / stomach / kidney | Salty, cold |
| | Sea horse | Liver / kidney | Warm | | Chrysanthemum indicum L. | Lung / liver | Bitter / hot, slight cold |
| | Donkey glue | Liver / kidney / lung | Flat | | Oldenlandia herbacea | Liver / spleen / stomach / large intestine / small intestine / kidney | Bitter / sweet, cold |
| | Curcuma rhizome | Liver / spleen | Warm | | Plantago seed | Liver / kidney / lung / small intestine | Sweet / light, cold |
| | Suberect spatholobus stem | Liver / kidney | Warm | | Plantago herb | Liver / kidney / lung / small intestine | Sweet / light, cold |
| Component (3) | Wild turmeric | Liver / spleen | Warm | | Wheat seed | Heart / liver | Sweet, slight cold |
| | Sparganium rhizome | Liver / spleen | Flat | | Feather cockscomb seed | Liver | Bitter, slight cold |
| | Luffa aegyptica Mill. | Liver / lung / stomach | Flat | | Chrysanthemum flower | Lung / liver | Sweet, slight bitter, slight cold |
| | Carthamus tinctorius | Heart / liver | Warm | | Dandelion herb | Liver / stomach | Bitter / sweet, cold |
| | Artemisia anomala | Heart / liver / kidney / lung / spleen | Warm | | Isatis tinctoria L. | Heart / liver / stomach | Salty / bitter, great cold |
| | Hairyveine agrimonia herb | Heart / liver / spleen / stomach | Flat | | Barbated skullcup herb | Liver / lung / stomach / kidney | Hot / bitter, slight cold |
| | Fortune windmillpalm petiole | Liver / lung / stomach | Flat | | Snowbellleaf tickclover herb | Liver / gall bladder / kidney / urinary bladder | Sweet / salty / light, slight cold |
| | Dalbergia odorifera | Heart / liver / spleen | Warm | | Turmeric | Heart / lung / liver / gall bladder | Hot / bitter, cold |
| | Dragon's blood | Heart / liver | Warm | | Garden burnet root | Liver / stomach / large intestine | Bitter / acid, slight cold |
| | Leech | Liver | Flat | | Crocus sativus | Heart / liver | Sweet, cold |
| | Panax notoginseng root | Liver / stomach | Warm | | | | |
| | Aizoon stonecrop herb | Heart / liver | Flat | | | | |
| | Ophicalcitum | Liver | Flat | | | | |
| | Lotus root node | Liver / lung / stomach | Flat | | | | |

Each herbal medicine of the component (1) will be briefly described. Longan aril is a semi-dried aril of Dimocarpus longan Lour. Of the family Sapindaceae. Common fenugreek seed is a dried seed of Trigonella foenum-graecum L. Lycium fruit is a dried fruit of Lycium chinense Mill. Or Lycium barbarum L. Songaria cynomorium herb is a dried whole grass of Cynomorium coccineum L. Black sesame is a dried seed of Sesamum indicum L. Dipsacus japonicus root is a dried root of Dipsacus asper Wall. Ex C.b.Clarke. Drynaria herb is a dried rhizome of Drynaria 16ortune (Kunze ex Mett.) J.Sm. Flatstem milkvetch seed is a dried seed of Astragalus complanatus R. Br. Black bean is a dried seed of Glycine max (L.) Merr. Stink-bug is a dried whole of Aspongopus chinensis Dallas. Sea horse is a dried whole of Hippocampus kelloggi Jordan et Snyder, Hippocampus histrix Kaup, Hippocampus kuda Bleeker., or Hippocampus trimaculatus Leach. Donkey glue is a dried glue made by heating and extracting the skin, bone, tendon or ligament of Equus asinus L. with water after the hair has been removed, removing the fat, and concentrating and drying the remaining extract.

Each herbal medicine of the component (2) will be briefly described. Ligustrum fruit is a dried fruit of Ligustrum lucidum Aiton. Oyster shell is a shell of Ostrea gigas Thunb. Yerbadetajo herb is a dried whole grass of Eclipta prostrata L. Mulberry fruit is a dried fruit of Morus alba L. or Morus australis Poir. Cassia seed is a dried seed of Cassia obtusifolia L. or Cassiatora L. Mentha herb is a dried aerial part of Mentha arvensis L. var. piperascens Malinv. Mulberry leaf is a dried leaf of Morus alba L. Gardenia fruit is a dried fruit of Gardenia jasminoides Ellis. Green tea leaf is a dried leaf of Camellia sinensis Kuntze. Tangle weed is a dried leaf of Saccharina japonica (Areschoug) C. E. Lane, C. Mayes, Oruehl et G. W. Saunders or s accharina religiosa (Miyabe) C. E. Lane, C. Mayes, Oruehl et G. W. Saunders or Saccharina ochotensis (Miyabe) C. E. Lane, C. Mayes, Oruehl et G. W. Saunders or Saccharina 17ortune17mi (Miyabe) C. E. Lane, C. Mayes, Oruehl et G. W. Saunders, or other plants of the genus Saccharina. Chrysanthemum indicum L. is a dried flower head of Chrysanthemum indicum L. Oldenlandia herbacea is a dried whole grass of Hedgotis diffusa WILLD. Plantago seed is a dried seed of Plantago asiatica L. Plantago herb is a dried whole grass in flowering time of Plantago asiatica L. Wheat seed is a dried seed of Triticum aestivum L. Feather cockscomb seed is a dried seed of Celosia argentae L. Chrysanthemum flower is a dried flower head of Chrysanthemum morifolium Ramat. Dandelion herb is a dried whole grass including the root of Taraxacum mongolicum Hand.-Mazz., Taraxacum sinicum Kitag., Taraxacum plantypecidum Diels, Taraxacum puseudo-albidum Kitag., Taraxacum cuspidatum Dahlst., or other plants of the genus Taraxacum. Isatis tinctoria L. is a dried leaf of Baphicacanthes cusia Bremek. Or Isatis tinctoria L. Barbated skullcup herb is a dried whole grass of Scutellaria barbata d. Don. Snowbellleaf tickclover herb is a dried whole grass of Desmodium styracifolium (Osbeck) Merr. Turmeric is a dried tuberous root of Curcuma longa L. or Curcuma aromatica Salisb. Garden burnet root is a dried root and rhizome of Sanguisorba officinalis L. Crocus sativus is a dried stigma of Crocus sativus L.

Each herbal medicine of the component (3) will be briefly described. Curcuma rhizome is a dried rhizome made usually by blanching and drying Curcuma zedoaria Roscoe. Suberect spatholobus stem is a dried stem of Spatholobus suberectus Dunn or Mucuna birdwoodiana Tutcher. Wild turmeric is a dried rhizome made usually by blanching and drying a rhizome of Curcuma longa L. as it is or after removing the cork layer. Sparganium rhizome is a dried rhizome of Sparganium stoloniferum Buchanan-Hamilton. Luffa aegyptica Mill. Is a dried net-like fiber of Luffa cylindrica Roem. Carthamus tinctorius is a dried tubular flower made by drying a tubular flower of Carthamus tinctorius L. as it is or after removing most of the yellow pigment, and sometimes pressing into a plate shape. Artemisia anomala is a dried whole grass of Siphonostegia chinensis Benth. Or Artemisia anomala s. Moore. Hairyveine agrimonia herb is a dried whole grass of Agrimonia 19ortun Ledeb. Fortune windmillpalm petiole is a dried leaf petiole of Trachycarpus 19ortune (Hook.f.) H.Wendl. Dalbergia odorifera is a dried xylem of Acronichia pedunculata (L) Miq. Dragon's blood is a dried resin of Daemonorops draco Blume. Leech is a dried whole of Whitmania pigra Whitman. Panax notoginseng root is a dried root of Panax notoginseng Feng Hwai Chen after removing the fine root. Aizoon stonecrop herb is a dried whole grass of Sedum aizoon L. with the root. Ophicalcitum is a marble Ophicalcite containing serpentine. Lotus root node is a dried node of a rhizome of Nelunbo nucifera Gaertn.

The herbal medicines of components (1), (2), and (3) have mild effects and can be taken as foods on a daily basis. Herbal medicines of each herbal medicine group can be arbitrarily combined, and particularly preferable combinations are shown in Table 2 below.

**[Table 2]**

| Combination | Component (1) | Component (2) | Component (3) |
|---|---|---|---|
| 1 | Longan aril, common fenugreek seed, Lycium fruit | Ligustrum fruit | Curcuma rhizome |
| 2 | Longan aril, common fenugreek seed | Oyster shell, yerbadetajo herb | Curcuma rhizome, Suberect spatholobus stem |
| 3 | Songaria cynomorium herb, Lycium fruit | Oyster shell | Carthamus tinctorius, Fortune windmillpalm petiole |
| 4 | Longan aril | Yerbadetajo herb, Ligustrum fruit | Wild turmeric, Suberect spatholobus stem |
| 5 | Black sesame, Dipsacus japonicus root | Yerbadetajo herb, mulberry fruit | Sparganium rhizome |
| 6 | Longan aril | Ligustrum fruit | Curcuma rhizome, Suberect spatholobus stem |
| 7 | Black sesame, Dipsacus japonicus root, songaria cynomorium herb | Ligustrum fruit | Wild turmeric |
| 8 | Lycium fruit, songaria cynomorium herb | Oyster shell, cassia seed | Sparganium rhizome |
| 9 | Longan aril, common fenugreek seed, Lycium fruit | Oyster shell | Curcuma rhizome |
| 10 | Longan aril | Oyster shell | Curcuma rhizome, Suberect spatholobus stem |
| 11 | Common fenugreek seed, Lycium fruit | Oyster shell, gardenia fruit | Curcuma rhizome, wild turmeric |
| 12 | Longan aril, Lycium fruit | Ligustrum fruit, mulberry fruit | Suberect spatholobus stem, Curcuma rhizome |
| 13 | Common fenugreek seed, Lycium fruit, black sesame | Ligustrum fruit, yerbadetajo herb | Suberect spatholobus stem, Carthamus tinctorius |

The herbal medicines as the components (1), (2), and (3), which are contained in the composition of the present invention, each may be a herbal medicine as it is, may be a herbal medicine finely shredded or crushed, or may be an extract of the herbal medicine. In the present specification, the extract of a herbal medicine is preferably an extract obtained by extracting the herbal medicine with a pharmaceutically acceptable aqueous solvent such as water (including hot water), ethanol, or a mixed solvent of water and ethanol. The herbal medicine extract may be prepared by a known extraction method, or may be a general soft extract, or an extract powder/extract granules.

The composition of the present invention is a composition for use in preventing, improving, or alleviating menstrual symptoms. There are three types of menstrual symptoms, that is, a premenstrual symptom, a symptom during menstruation, and a symptom that occurs from the premenstrual period to the menstrual period. The premenstrual symptom mainly refers to premenstrual syndrome (PMS), and the PMS refers to mental and physical symptoms that begin 3 to 10 days before menstruation, and decreases or disappears with the onset of menstruation. The symptom during menstruation mainly refers to dysmenorrhea or menstrual pain. The dysmenorrhea or menstrual pain is a pathological symptom that occurs with menstruation during the menstrual period, and as such a pathological symptom, lower abdominal pain, low back pain, abdominal bloating, nausea, headache, fatigue, weakness, anorexia, irritation, diarrhea, and depression are most frequently observed in this order. The symptom that occurs from the premenstrual period to the menstrual period mainly refers to perimenstrual syndrome (PEMS), and symptoms of the PEMS include psychiatric symptoms such as irritation, lack of motivation, heightened anxiety, and depression, and social symptoms such as wanting to be alone, and disliking menstruation.

The composition of the present invention is a composition that can be used for preventing, improving, or alleviating various menstrual symptoms by mixing herbal medicines having specific medicinal benefit coverages and nature tastes. That is, the component (1) acts on the liver relating to the gynecological system to increase the blood, the component (3) activates the circulation of blood, and the component (2) balances the cold/warm to regulate the life force and blood (circulation of blood) of women, and as a result of which the composition can prevent, improve, or alleviate various menstrual symptoms. In particular, the composition is effective for menstrual symptoms such as menstrual pain, lower abdominal pain, breast strain or pain, abnormality in menstrual bleeding amount (heavy or low menstrual flow), abnormality in vaginal discharge (a lot of or little vaginal discharge, sticky vaginal discharge, etc.), anemia, unusual increase in appetite, dyspeptic symptom, feeling of cold, swelling, headache, constipation, diarrhea, abdominal bloating, and symptoms in the genital area and the surrounding area thereof, and, for example, the composition is effective for at least one symptom, at least three symptoms, at least five symptoms, at least seven symptoms, at least nine symptoms, at least 11 symptoms, at least 13 symptoms, or all the symptoms selected from these menstrual symptoms. In this regard, the unusual increase in appetite means a phenomenon in which an increase in appetite peculiar to the menstrual period, which is different from that in the usual time, is observed, and the effect of fluctuations in hormonal balance has been pointed out. The symptoms in the genital area and the surrounding area thereof mean pain, itching, inflammation, creepy-crawly feeling, smell, and the like in the genital area and the surrounding area thereof (so-called female genital area).

Further, the composition of the present invention can prevent, improve, or alleviate at least one symptom (for example, at least two symptoms, at least three symptoms, at least four symptoms, at least five symptoms, at least six symptoms, at least seven symptoms, at least eight symptoms, or at least nine or more symptoms) selected from the symptoms such as bad complexion/sallowness, unhealthy complexion, dull skin/dark circles under the eyes, acne/pimples, difficulty in healing wounds/bruises, rough skin/itchy skin, decreased libido, stiff shoulders, low back pain, menopausal symptom, chronic fatigue, hair loss, tinnitus, dry eye, eye fatigue, emotional lability (emotional mood swing, easy to get angry, etc.), dysphoria (easy to feel depressed, etc.)/depressive symptom, sleepiness, difficulty falling asleep, light sleep, ease of catching a cold, and redness of the palm. These symptoms/conditions are not necessarily menstrual symptoms, but are symptoms related to problems peculiar to women (related to female hormones) or circulation of blood, and it is considered that the composition of the present invention can prevent, improve, or alleviate extensively also the whole symptoms related to the problems peculiar to women or the circulation of blood by regulating the life force and blood (blood circulation). The whole symptoms related to the problems peculiar to women or the circulation of blood can be classified into: beauty-related symptoms including bad complexion/sallowness, unhealthy complexion, dull skin/dark circles under the eyes, acne/pimples, difficulty in healing wounds/bruises, rough skin/itchy skin, and the like; menopause-related symptoms including decreased libido, stiff shoulders, low back pain, menopausal symptom, chronic fatigue, hair loss, tinnitus, dry eye, eye fatigue, and the like; mental-related symptoms including emotional lability, dysphoria/depressive symptom, sleepiness, difficulty falling asleep, light sleep, and the like; and other related symptoms including ease of catching a cold, redness of the palm, and the like.

The mixing amounts of herbal medicines as the components (1), (2), and (3) contained in the composition of the present invention may be each appropriately set according to various conditions of the person who ingests the composition, such as body weight, age, and symptoms, may be each an amount sufficient to prevent, improve, or alleviate menstrual symptoms, and are each prescribed by the weight of herbal medicine per daily dose (in a case of an extract, the weight of herbal medicine used (herbal medicine equivalent weight)) (g/day). The composition may be ingested once a day, or may be ingested in several divided doses, for example, 2, 3 or 4 divided doses. In a case of ingesting the composition in several divided doses, the composition should be ingested so that the total doses are the daily dose.

The daily dose of each herbal medicine includes, as the component (1) 3 to 12 g of Longan aril, 3 to 9 g of common fenugreek seed, 3 to 9 g of Lycium fruit, 3 to 9 g of songaria cynomorium herb, 9 to 30 g of black sesame, 9 to 15 g of Dipsacus japonicus root, 9 to 15 g of Drynaria herb, 9 to 15 g of flatstem milkvetch seed, 30 to 60 g of black bean, 3 to 6 g of stink-bug, 3 to 9 g of sea horse, and 6 to 15 g of donkey glue; as the component (2) 2 to 15 g of Ligustrum fruit, 2 to 30 g of oyster shell, 2 to 15 g of yerbadetajo herb, 3 to 30 g of mulberry fruit, 2 to 15 g of cassia seed, 1.5 to 6 g of mentha herb, 3 to 9 g of mulberry leaf, 3 to 9 g of gardenia fruit, 3 to 15 g of green tea leaf, 10 to 15 g of tangle weed, 9 to 15 g of Chrysanthemum indicum L., 15 to 60 g of Oldenlandia herbacea, 3 to 9 g of plantago seed, 9 to 15 g of plantago herb, 30 to 60 g of wheat seed, 3 to 15 g of feather cockscomb seed, 6 to 12 g of chrysanthemum flower, 6 to 30 g of dandelion herb, 6 to 15 g of Isatis tinctoria L., 15 to 30 g of barbated skullcup herb, 15 to 30 g of snowbellleaf tickclover herb, 3 to 9 g of turmeric, 9 to 15 g of garden burnet root, and 1 to 3 g of Crocus sativus; and as the component (3) 1.5 to 9 g of Curcuma rhizome, 3 to 60 g of Suberect spatholobus stem, 1.5 to 9 g of wild turmeric, 1.5 to 9 g of Sparganium rhizome, 3 to 15 g of Luffa aegyptica Mill., 3 to 15 g of Carthamus tinctorius, 9 to 60 g of Artemisia anomala, 9 to 60 g of Hairyveine agrimonia herb, 6 to 15 g of Fortune windmillpalm petiole, 3 to 6 g of Dalbergia odorifera, 1 to 1.5 g of dragon's blood, 3 to 6 g of leech, 3 to 9 g of Panax notoginseng root, 15 to 30 g of Aizoon stonecrop herb, 3 to 9 g of Ophicalcitum, and 10 to 30 g of lotus root node. In a case where multiple herbal medicines are used as the component (1), (2), or (3), the daily dose of each herbal medicine may be appropriately reduced.

The composition of the present invention is preferably a food composition or a pharmaceutical composition. The food composition may be in any possible form as food. Examples of the form include: a milk product; a fermented food (yogurt, cheese, et al.); beverages (coffee, juice, a soft drink such as a tea beverage, a carbonated beverage, a milk beverage, a lactic acid bacteria beverage, a beverage containing lactic acid bacteria, a yogurt drink, and liquors such as Japanese rice wine, fruit wine, and Western liquor); spreads (custard cream, et al.); pastes (fruit paste, et al.); Western confectionery (donuts, pies, cream puffs, chewing gum, candy, jelly, cookies, cake, chocolate, pudding, et al.); Japanese confectionery (Daifuku (soft round rice cake stuffed with sweet bean jam), rice cake, sweet bun (bun with a bean-jam filling), sponge cake, Anmitsu (agar jelly served with red bean paste and brown sugar syrup), Yokan (sweet bean jelly), et al.); ice confectionery (ice cream, popsicles, sherbet, et al.); foods (curry, Gyudon (a bowl of rice topped with boiled beef and onions), porridge of rice and vegetables, rice gruel, miso (fermented soybean paste) soup, soup, meat sauce, pasta, Japanese pickled vegetables, jam, etc.); and condiments (dressing, flavor enhancer, Furikake (dried food sprinkling over rice), broth, et al.) Such a food composition can be served as a dish at a restaurant, and can also be provided in a form of general distribution by taking a form of prepared food, retort pouch food, a form of frozen food, another packaging form or the like of a general food composition.

In addition to the components (1), (2), and (3) as the active components, the food composition in the above various forms may contain a component that is acceptable as food, as long as it does not affect the action of the active components. Examples of the component include various general foodstuffs, starch, gelatin, vegetable fat and oil, animal fat and oil, processed fat and oil or the like, carboxymethyl cellulose, gum arabic, locust bean gum, gum tragacanth, karaya gum, konjac glucomannan, xanthane gum, agar, carrageenan, polyacrylic acid, carob bean gum, alginic acid, a gellant such as a cyclodextrin, a seasoning, a sweetener, a spice, a colorant, a preservative, an antioxidant, water, an algefacient, a binder, a sweetener, a disintegrant, a lubricant, a colorant, flavor, a stabilizer, an antiseptic, a sustained release regulator, a surfactant, a solubilizer, and a wetting agent.

The food composition of the present invention may appropriately arbitrarily mix a functional food material for the purpose of further exerting a complex effect. As the functional food material, for example, various kinds of herbal medicines other than the components (1), (2), and (3), various kinds of probiotics, various kinds of oligosaccharides, nutrient components such as vitamins and minerals, flavonoids, quinones, polyphenols, amino acid, nucleic acid, essential fatty acid, and other known functional food materials may be appropriately arbitrarily mixed.

The production method of food compositions in various forms is not particularly limited, and a known method can be used appropriately. Further, a component or treatment capable of reducing the peculiar taste and smell of a herbal medicine may be added or performed.

The food composition of the present invention may also be a food for the purpose of preservation of health, health maintenance, health promotion, and the like, for example, a health food, a food with function claims, a nutritional supplementary food, a supplement, a food for specified health uses, a food with nutrient function claims, or the like. In a case of being a supplement, the food composition of the present invention may be in a form of, for example, tablets, capsules, granules, a liquid, a powder, a drink, jelly, or the like.

The contents of components (1), (2), and (3) as active components in the food composition of the present invention are not particularly limited, and vary depending on the form of the food composition. For example, in a case where the food composition is in a form of tablets/capsules/granules/a liquid/a powder/a supplement such as stick jelly, the total weight of each herbal medicine in the food composition can be mentioned to be typically 10 to 80% by mass of the food composition. Further, in a case where the form of the food composition is a form of various kinds of beverages/jelly, or a form of general foods such as foods/retort pouch foods, the total weight of each herbal medicine in the food composition can be mentioned to be typically 0.05 to 25% by mass of the food composition. Considering the taste and the amount, it is preferable to minimize the mixing amounts of the active components in a food composition, and in that case, it is preferable to use an extract of a herbal medicine rather than mixing the herbal medicine as it is. Since the herbal medicine contained in the food composition of the present invention has a mild effect when usually used as a food, the intake is not particularly limited. If the intake of the food composition of the present invention is an amount that includes the daily doses of the above-described herbal medicines, the desired effects of preventing, improving, or alleviating menstrual symptoms can be obtained.

The pharmaceutical composition of the present invention may be in a form of a drink, syrup, jelly, a lyophilized powder, tablets, capsules, pills, a powder, fine granules, granules, or the like, and may contain in addition to the components (1), (2), and (3) as the active components, for example, a pharmaceutically acceptable component, for example, an excipient, a binder, a lubricant, a disintegrant, an emulsifier, a surfactant, a base, a dissolution aid, or a suspending agent.

The composition of the present invention may also be used in combination with other compositions. For example, by using the composition of the present invention in combination with an over-the-counter drug that is used to treat menstrual symptoms, for example, a nonsteroidal antiinflammatory agent, a low-dose oral contraceptive, or the like, discomfort symptoms can be eliminated with minimal use of the over-the-counter drug.

The timing of the ingestion of the composition of the present invention is not particularly limited, and in a case where the ingestion is once a day, the composition may be taken at any time such as after waking up, before meal, between meals, after meal, or before going to bed, and in a case where the ingestion is several times a day, the composition may be taken at two, three, or four or more of timings of after waking up, before meal, between meals, after meal, and before going to bed. Further, the composition may be ingested together with meal. The food composition of the present invention may be continuously ingested for 1 week or more, preferably 2 weeks or more, and more preferably 4 weeks or more.

Hereinafter, examples will be given in order to describe the present invention further in detail. However, the present invention should not be limited at all to these examples.

### EXAMPLES

### <Example 1>

As the raw material herbal medicines, Longan aril, common fenugreek seed, Lycium fruit, Ligustrum fruit, and Curcuma rhizome were mixed in a proportion of 6 : 3 : 3 : 4.5 : 3 in terms of weight ratio, the mixture was boiled and extracted in water, the filtration, concentration, and sterilization were performed, dextrin in an adequate amount was added to the obtained product, and then the resultant product was dried with a spray dryer to obtain a mixed plant extract powder. In the obtained extract, 6.3 g of extract powder equivalent to 6 g of Longan aril, 3 g of common fenugreek seed, 3 g of Lycium fruit, 4.5 g of Ligustrum fruit, and 3 g of Curcuma rhizome as the raw material herbal medicines, was used as a food composition for one day.

### <Example 2>

As the raw material herbal medicines, songaria cynomorium herb, Lycium fruit, oyster shell, Carthamus tinctorius, and Fortune windmillpalm petiole were mixed in a proportion of 3 : 3 : 4.5 : 3 : 6 in terms of weight ratio, the mixture was boiled and extracted in water, the filtration, concentration, and sterilization were performed, dextrin in an adequate amount was added to the obtained product, and then the resultant product was dried with a spray dryer to obtain a mixed plant extract powder. In the obtained extract, 6.0 g of extract powder equivalent to 3 g of songaria cynomorium herb, 3 g of Lycium fruit, 4.5 g of oyster shell, 3 g of Carthamus tinctorius, and 6 g of Fortune windmillpalm petiole as the raw material herbal medicines, was used as a food composition for one day.

### <Example 3>

As the raw material herbal medicines, Longan aril, yerbadetajo herb, Ligustrum fruit, wild turmeric, and Suberect spatholobus stem were mixed in a proportion of 3 : 3 : 4.5 : 3 : 9 in terms of weight ratio, the mixture was boiled and extracted in water, the filtration, concentration, and sterilization were performed, dextrin in an adequate amount was added to the obtained product, and then the resultant product was dried with a spray dryer to obtain a mixed plant extract powder. In the obtained extract,7.5 g of extract powder equivalent to 3 g of Longan aril, 3 g of yerbadetajo herb, 4.5 g of Ligustrum fruit, 3 g of wild turmeric, and 9 g of Suberect spatholobus stem as the raw material herbal medicines, was used as a food composition for one day.

### <Comparative Example 1>

As the raw material herbal medicines, cassia seed, mulberry fruit, Houttuynia herb, and gardenia fruit were mixed in a proportion of 3 : 3 : 3 : 3 in terms of weight ratio, the mixture was boiled and extracted in water, the filtration, concentration, and sterilization were performed, dextrin in an adequate amount was added to the obtained product, and then the resultant product was dried with a spray dryer to obtain a mixed plant extract powder. In the obtained extract, 5.4 g of extract powder equivalent to 3 g of cassia seed, 3 g of mulberry fruit, 3 g of Houttuynia herb, and 3 g of gardenia fruit was used as a food composition for one day.

### <Comparative Example 2>

As the raw material herbal medicines, Longan aril, Fortune windmillpalm petiole, and mung bean were mixed in a proportion of 3 : 6 : 15 in terms of weight ratio, the mixture was boiled and extracted in water, the filtration, concentration, and sterilization were performed, dextrin in an adequate amount was added to the obtained product, and then the resultant product was dried with a spray dryer to obtain a mixed plant extract powder. In the obtained extract, 6.0 g of extract powder equivalent to 3 g of Longan aril, 6 g of Fortune windmillpalm petiole, and 15 g of mung bean as the raw material herbal medicines, was used as a food composition for one day.

List of the mixing amount, medicinal benefit coverage, and five natures of each of the herbal medicines in Examples 1 to 3 and Comparative Examples 1 and 2 is shown in Table 3.

**[Table 3]**

| | Component (1) | | | Component (2) | | Component (3) | | Other components | |
|---|---|---|---|---|---|---|---|---|---|
| Example 1 | Longan aril | Common fenugreek seed | Lycium fruit | Ligustrum fruit | | Curcuma rhizome | | | |
| Daily dose (g) | 6 | 3 | 3 | 4.5 | | 3 | | | |
| Medicinal benefit coverage | Heart / spleen | Liver / kidney | Liver / kidney / lung | Liver / kidney | | Liver / spleen | | | |
| Five natures | Flat | Great warm | Flat | Cool | | Warm | | | |
| Example 2 | Songaria cynomorium herb | Lycium fruit | | Oyster shell | | Carthamus tinctorius | Fortune windmillpalm petiole | | |
| Daily dose (g) | 3 | 3 | | 4.5 | | 3 | 6 | | |
| Medicinal benefit coverage | Liver / kidney | Liver / kidney / lung | | Liver / kidney | | Heart / liver | Liver / lung / large intestine | | |
| Five natures | Warm | Flat | | Slight cold | | Warm | Flat | | |
| Example 3 | Longan aril | | | Yerbadetajo herb | Ligustrum fruit | Wild turmeric | Suberect spatholobus stem | | |
| Daily dose (g) | 3 | | | 3 | 4.5 | 3 | 9 | | |
| Medicinal benefit coverage | Heart / spleen | | | Liver / kidney | Liver / kidney | Liver / spleen | Liver / kidney | | |
| Five natures | Flat | | | Cold | Cool | Warm | Warm | | |
| Comparative Example 1 | | | | Mulberry fruit | Cassia seed | | | Houttuynia herb | Gardenia fruit |
| Daily dose (g) | | | | 3 | 3 | | | 3 | 3 |
| Medicinal benefit coverage | | | | Heart / liver / kidney | Liver / kidney | | | Kidney / lung | Heart / liver / lung |
| Five natures | | | | Cold | Slight cold | | | Slight cold | Cold |
| Comparative Example 2 | Longan aril | | | | | Fortune windmillpalm petiole | | Mung bean | |
| Daily dose (g) | 3 | | | | | 6 | | 15 | |
| Medicinal benefit coverage | Heart / spleen | | | | | Liver / lung / large intestine | | Heart / stomach | |
| Five natures | Flat | | | | | Flat | | Cold | |

### <Test Example 1>

### (Evaluation test 1 of effect on physical disorder including menstrual symptom)

A randomized, double-blind, parallel-group controlled trial was conducted on 110 Japanese women aged 20 or more and 49 or less who had been aware of menstrual symptoms or daily physical problems, and the effects of the present invention were investigated. Each of 54 subjects ingested the extract powder of Example 1 continuously for 4 weeks (28 days) at a daily dose of 2.1 g once a day between meals after dissolving the powder in about 150 cc of lukewarm water. Further, each of the other 56 subjects ingested the extract powder of Comparative Example 1 continuously for 4 weeks (28 days) at a daily dose of 1.8 g once a day between meals after dissolving the powder in about 150 cc of lukewarm water. Discomfort questionnaires were conducted for all the subjects before the ingestion and at the completion of the ingestion for 4 weeks, and the degree of improvement in the symptoms was evaluated. In the discomfort questionnaires, for each symptom group listed in Table 4, scores of 5 levels: "very worried (1)", "slightly worried (2)", "cannot say (3)", "hardly bothered (4)", and "not bothered at all (5)" were set, and the degree of improvement was evaluated by the fluctuation in the scores for each symptom before and after the ingestion. More specifically, for each questionnaire item (discomfort symptom), the proportion of the subjects of which each score of the questionnaire at the completion of the ingestion was 4 or 5 among the subjects of which each score of the questionnaire before the ingestion was 1 or 2 was taken as the improvement rate, and it was determined that the extract had an effect on the improvement of the discomfort symptom having an improvement rate of 30% or more.

### (Results)

The results are shown in Table 4. From Table 4, by the ingestion of the extract of Example 1, all of the menstrual symptoms such as menstrual pain, lower abdominal pain, breast strain or pain, abnormality in menstrual bleeding amount, abnormality in vaginal discharge, anemia, unusual increase in appetite, dyspeptic symptom, feeling of cold, swelling, headache, constipation/diarrhea/abdominal bloating, and symptoms in the genital area and the surrounding area thereof (female genital area) were improved, and further, bad complexion/sallowness, unhealthy complexion, dull skin/dark circles under the eyes, acne/pimples, difficulty in healing wounds/bruises, rough skin/itchy skin, decreased libido, low back pain, menopausal symptoms, tinnitus, emotional lability (emotional mood swing), dysphoria/depressive symptom, ease of catching a cold, and symptoms related to problems peculiar to women (related to female hormones) such as redness of the palm or circulation of blood were also improved. In this regard, also by the ingestion of the extract of Comparative Example 1, some of the menstrual symptoms were improved, but the improvement in the menstrual symptoms and the whole symptoms related to problems peculiar to women or circulation of blood as in Example 1 was not observed. This is considered to be a limited effect of the herbal medicine of the component (2) contained in Comparative Example 1.

**[Table 4]**

| Group | Item | Bad complexion, sallowness, unhealthy complexion | Dull skin (face) and / or dark circles under the eyes | Ease of acne / pimples | Difficulty in healing wounds and / or bruises | Rough skin / itchy skin | Menstrual pain | Lower abdominal pain | Breast pain / breast strain | Abnormality in menstrual bleeding amount (heavy / low menstrual flow, etc.) | Abnormality in vaginal discharge (large amount / sticky vaginal discharge, etc.) | Anemia (there is dizziness / orthostatic dizziness / grogginess) | Unusual increase in appetite | Dyspeptic symptoms (stomach discomfort / nausea / heartburn) | Feeling of cold (cold limbs / low body temperature) | Swelling of face and limbs | Headache |
|---|---|---|---|---|---|---|---|---|---|---|---|---|---|---|---|---|---|
| Example 1 | Improvement rate (%) | 32 | 32 | 46 | 30 | 36 | 39 | 44 | 44 | 32 | 42 | 42 | 35 | 67 | 46 | 38 | 39 |
| | Improvement | ○ | ○ | ○ | ○ | ○ | ○ | ○ | ○ | ○ | ○ | ○ | ○ | ○ | ○ | ○ | ○ |
| Comparative Example 1 | Improvement rate (%) | 17 | 24 | 22 | 26 | 40 | 42 | 44 | 66 | 31 | 27 | 29 | 44 | 35 | 38 | 27 | 35 |
| | Improvement | | | | | ○ | ○ | ○ | ○ | ○ | | | ○ | ○ | ○ | | ○ |

| Group | Item | Constipation (constipation / abdominal bloating / diarrhea, etc.) | Symptoms in the female genital area (itching / creepy-crawly feeling / smell, etc.) | Decreased libido (frigidity / coital pain) | Low back pain | Menopausal symptoms (hot flashes / sweating / palpitations / shortness of breath, etc.) | Chronic fatigue | Excessive hair loss / increased hair loss | Tinnitus | Dry eye | Eye fatigue | Emotional mood swing (depressed, easy to get angry) | Mental anxiety (easy to feel depressed) | Sleepiness | Difficulty falling asleep / light sleep / waking up during the night | Ease of catching a cold | Redness of the palm |
|---|---|---|---|---|---|---|---|---|---|---|---|---|---|---|---|---|---|
| Example 1 | Improvement rate (%) | 46 | 46 | 50 | 32 | 46 | 19 | 24 | 50 | 14 | 15 | 36 | 33 | 26 | 32 | 50 | 50 |
| | Improvement | ○ | ○ | ○ | ○ | ○ | | | ○ | | | ○ | ○ | | | ○ | ○ |
| Comparative Example 1 | Improvement rate (%) | 37 | 26 | 8 | 24 | 17 | 20 | 27 | 47 | 22 | 20 | 27 | 29 | 18 | 27 | 71 | 25 |
| | Improvement | ○ | | | | | | | ○ | | | | | | | | |

### <Test Example 2>

### (Evaluation test 2 of effect on physical disorder including menstrual symptom)

A single-blind controlled before-after trial was conducted with the following design, and the effects of the present invention were investigated. The single-blind controlled before-after trial was conducted on 13 Japanese women aged 19 or more and 47 or less who had been aware of menstrual symptoms or daily physical problems, and the subjects ingested the extract powder of Example 3 continuously for 4 weeks (28 days) at a daily dose of 2.5 g once a day between meals after dissolving the powder in about 150 cc of lukewarm water. Discomfort questionnaires were conducted for all the subjects before the ingestion and at the completion of the ingestion for 4 weeks, and the degree of improvement in the symptoms was evaluated. In the discomfort questionnaires before the ingestion, for each symptom group listed in Table 5, scores of 3 levels: "extremely worried (1)", "slightly worried (2)", and "not worried (3)" were set, and the subjects reporting to have symptoms before the ingestion were determined. In the discomfort questionnaires at the completion of the ingestion, for each symptom group listed in Table 5, scores of 5 levels: "very improved (1)", "improved (2)", "unchanged (3)", "impaired (4)", and "very impaired (5)" were set, and the degree of improvement was evaluated for each symptom before and after the ingestion. A washout period of 4 weeks or more after the completion of the ingestion was set, and then, similarly, the same subjects ingested the extract powder of Example 1 continuously for 4 weeks at a daily dose of 2.1 g once a day, and discomfort questionnaires were conducted for all the subjects before the ingestion and at the completion of the ingestion. Similarly after that, the same subjects ingested the extract powder of Comparative Example 2 continuously for 4 weeks at a daily dose of 2.0 g once a day after a washout period of 4 weeks or more was set, and then the extract powder of Comparative Example 1 continuously for 4 weeks at a daily dose of 1.8 g once a day after a washout period of 4 weeks or more was set, and discomfort questionnaires were conducted for all the subjects before the ingestion and at the completion of the ingestion. Further, separately, different subjects, that is, 13 Japanese women aged 24 or more and 53 or less who had been aware of menstrual symptoms or daily physical problems ingested the extract powder of Example 2 continuously for 4 weeks at a daily dose of 2.0 g once a day, and discomfort questionnaires were conducted for all the subjects before the ingestion and at the completion of the ingestion.

For the indicator for evaluating the degree of improvement of each symptom, the proportion of the subjects of which each score of the questionnaire at the completion of the ingestion was 1 or 2 to the subjects (subjects reporting to have symptoms) of which each score of the questionnaire before the ingestion was 1 or 2 was used as the improvement rate for each questionnaire item (discomfort symptom). In Test Example 2, the number of subjects was limited as compared with the subjects in Test Example 1, and it was difficult to quantitatively compare the improvement rate. Thus, for the purpose of qualitative comparison of the degree of improvement, it was determined that the extract had an effect (o) on the improvement of the discomfort symptom of which the improvement rate had been a positive value. In this regard, the number of subjects in Table 5 was the number of subjects who complained of discomfort symptoms (number of subjects reporting to have symptoms) before the start of the trial, and the items of discomfort symptoms in which the number of subjects reporting to have symptoms was 20% or less of the total number of subjects were excluded from the evaluation as invalid data (-).

### (Results)

The results are shown in Table 5. From Table 5, by the ingestion of the extract of Comparative Example 1 or Comparative Example 2, some of menstrual symptoms such as menstrual pain, breast strain or pain, abnormality in menstrual bleeding amount, abnormality in vaginal discharge amount, cold limbs, and constipation were improved, and acne/pimples were also improved. This is considered to be a limited effect of the herbal medicine of component (2) contained in Comparative Example 1, or the herbal medicines of components (1) and (3) contained in Comparative Example 2. Meanwhile, by the ingestion of the extracts of Examples 1 to 3, in addition to the improvement in symptoms observed by the ingestion of the extracts of Comparative Examples 1 and 2, menstrual symptoms such as abnormality in vaginal discharge stickiness, diarrhea, abdominal bloating, and symptoms in the genital area and the surrounding area thereof, which were not improved by the ingestion of the extracts of Comparative Examples 1 and 2, were also improved. Further, by the ingestion of the extracts of Examples 1 to 3, the whole symptoms related to the problems peculiar to women or circulation of blood, such as bad complexion/sallowness, unhealthy complexion, rough skin/itchy skin, stiff shoulders/low back pain, chronic fatigue, hair loss, tinnitus, dry eye, eye fatigue, sleepiness, difficulty falling asleep, and light sleep were extensively improved.

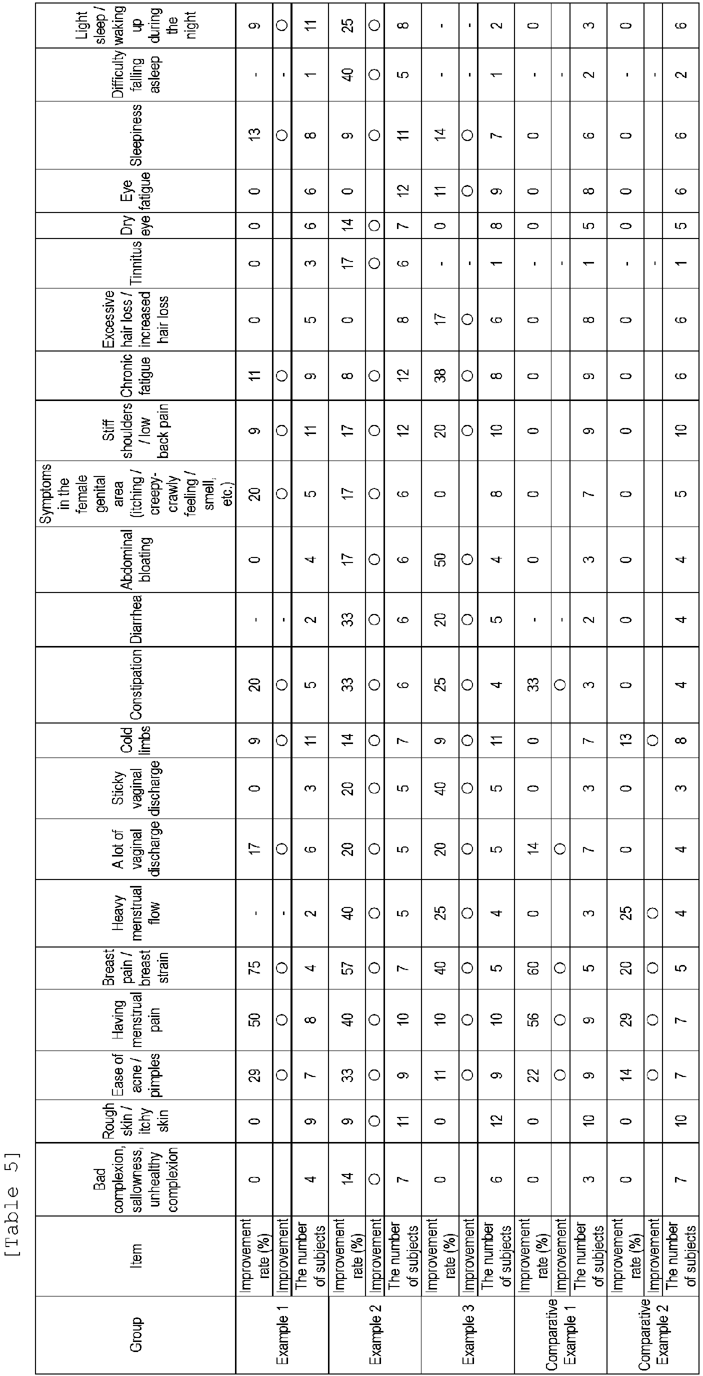

From the results of Test Examples 1 and 2, by the ingestion of the extract of Comparative Example 1 containing component (2) but not containing components (1) and (3), or the extract of Comparative Example 2 containing components (1) and (3) but not containing component (2), the effects on the prevention, improvement, or alleviation were not able to be confirmed only in some of the menstrual symptoms, but in contrast, by the ingestion of the extract of Example 1, 2, or 3 containing components (1), (2), and (3), which is the composition of the present invention, the effects on the prevention, improvement, or alleviation were able to be extensively confirmed in menstrual symptoms and the whole symptoms related to the problems peculiar to women or circulation of blood.

## Claims

1. A composition, comprising as active components the following components (1), (2), and (3):
component (1): at least one kind of herbal medicine selected from the group consisting of Longan aril, common fenugreek seed, Lycium fruit, songaria cynomorium herb, black sesame, Dipsacus japonicus root, Drynaria herb, flatstem milkvetch seed, black bean, stink-bug, sea horse, and donkey glue;
component (2): at least one kind of herbal medicine selected from the group consisting of Ligustrum fruit, oyster shell, yerbadetajo herb, mulberry fruit, cassia seed, mentha herb, mulberry leaf, gardenia fruit, green tea leaf, tangle weed, Chrysanthemum indicum L., Oldenlandia herbacea, plantago seed, plantago herb, wheat seed, feather cockscomb seed, chrysanthemum flower, dandelion herb, Isatis tinctoria L., barbated skullcup herb, snowbellleaf tickclover herb, turmeric, garden burnet root, and Crocus sativus; and
component (3): at least one kind of herbal medicine selected from the group consisting of Curcuma rhizome, Suberect spatholobus stem, wild turmeric, Sparganium rhizome, Luffa aegyptica Mill., Carthamus tinctorius, Artemisia anomala, Hairyveine agrimonia herb, Fortune windmillpalm petiole, Dalbergia odorifera, dragon's blood, leech, Panax notoginseng root, Aizoon stonecrop herb, Ophicalcitum, and lotus root node.

2. The composition according to claim 1, wherein the component (2) is at least one kind of herbal medicine selected from the group consisting of Ligustrum fruit, oyster shell, yerbadetajo herb, mulberry fruit, and cassia seed.

3. The composition according to claim 1 or 2,
wherein the composition is for use in preventing, improving, or alleviating menstrual symptoms.

4. The composition according to any one of claims 1 to 3, wherein the menstrual symptoms are at least one selected from the group consisting of menstrual pain, lower abdominal pain, breast strain or pain, abnormality in menstrual bleeding amount, abnormality in vaginal discharge, anemia, unusual increase in appetite, dyspeptic symptom, feeling of cold, swelling, headache, constipation, diarrhea, abdominal bloating, and symptoms in the genital area and the surrounding area thereof.

5. The composition according to any one of claims 1 to 4, wherein the composition is a food composition or a pharmaceutical composition.
